(19) Europäisches Patentamt
European Patent Office
Office européen des brevets

(11) EP 2 842 553 A1

(12) **EUROPEAN PATENT APPLICATION**
published in accordance with Art. 153(4) EPC

(43) Date of publication:
**04.03.2015 Bulletin 2015/10**

(21) Application number: **13782289.6**

(22) Date of filing: **26.04.2013**

(51) Int Cl.:
***A61K 31/16*** *(2006.01)* ***A61K 9/08*** *(2006.01)*
***A61K 9/20*** *(2006.01)* ***A61K 9/70*** *(2006.01)*
***A61K 31/231*** *(2006.01)* ***A61K 31/265*** *(2006.01)*
***A61K 47/40*** *(2006.01)* ***A61P 19/02*** *(2006.01)*
***A61P 29/00*** *(2006.01)*

(86) International application number:
**PCT/JP2013/062374**

(87) International publication number:
**WO 2013/161994 (31.10.2013 Gazette 2013/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.04.2012 JP 2012103305**

(71) Applicants:
- **Nippon Zoki Pharmaceutical Co., Ltd.**
  **Osaka-shi, Osaka 541-0046 (JP)**
- **Nagoya Industrial Science Research Institute**
  **Nagoya-shi**
  **Aichi 460-0008 (JP)**

(72) Inventors:
- **IINUMA, Munekazu**
  **Gifu-shi**
  **Gifu 501-1196 (JP)**
- **FURUKAWA, Shoei**
  **Gifu-shi**
  **Gifu 501-1196 (JP)**
- **NAIKI, Mitsuru**
  **Kato-shi**
  **Hyogo 673-1461 (JP)**
- **MATSUMOTO, Tomonori**
  **Kato-shi**
  **Hyogo 673-1461 (JP)**
- **SAWADA, Kazuyoshi**
  **Kato-shi**
  **Hyogo 673-1461 (JP)**

(74) Representative: **Hoffmann Eitle**
**Patent- und Rechtsanwälte PartmbB**
**Arabellastraße 30**
**81925 München (DE)**

(54) **ANALGESIC**

(57) An object of the present invention is to provide an analgesic effective in a prophylactic or a therapy for various pain diseases. The present invention is to provide an analgesic containing at least one member selected from a trans-2-decenoic acid derivative represented by the formula (1) and a pharmaceutically acceptable salt thereof as an active ingredient:

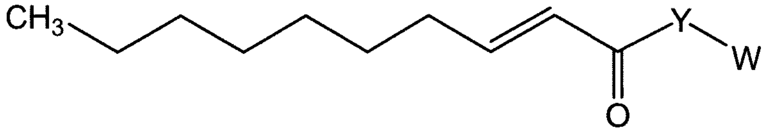

(1)

(In the formula, Y is -O-, -NR- or -S-, R is hydrogen atom, alkyl group, dialkylaminoalkyl group or the like and W is a substituent such as dialkylaminoalkyl group). The analgesic of the present invention containing the compound as an active ingredient is highly useful as a prophylactic or therapeutic agent for various pain diseases such as the pain caused by osteoarthritis (e.g., knee osteoarthritis and hip osteoarthritis).

EP 2 842 553 A1

**Description**

[Technical Field]

The present invention relates to a novel pharmaceutical use of a trans-2-decenoic acid derivative or a pharmaceutically acceptable salt thereof and, more particularly, it relates to an analgesic containing at least one member selected from a trans-2-decenoic acid derivative having a structure described below and a pharmaceutically acceptable salt thereof as an active ingredient.

[Background Art]

The present invention relates to an analgesic containing at least one member selected from a trans-2-decenoic acid derivative and a pharmaceutically acceptable salt thereof described below as an active ingredient. Patent Document 1 discloses that a $C_8$ or $C_{10\sim12}$ fatty acids as well as ester thereof has/have a neurotrophic factor-like activity. However, the compounds mentioned in Patent Document 1 have a different chemical structure from the compounds which is an active ingredient of the analgesic in the present invention. Additionally, with regard to the agent having a neurotrophic factor-like activity in the Patent Document 1, there is a mere description that it is useful as a prophylactic/improving agent for neurodegenerative disease such as Alzheimer disease or Parkinson disease and for mental disease such as depression or anxiety disorder (neurosis) and there is no description therein at all that an analgesic action as in the present invention is available. Meanwhile, (*E*)-*N*-isobutyl dec-2-enamide (compound 18), (*E*)-*N*-phenyl dec-2-enamide (compound 28), (*E*)-*N*-phenethyl dec-2-enamide (compound 29) and (*E*)-*N,N*-diethyl dec-2-enamide (compound 31) which are a compound in accordance with the analgesic of the present invention are disclosed in Patent Document 2, Non-Patent Document 1, Non-Patent Document 2, or Non-Patent Document3, respectively. However, Patent Document 2 relates to an action for controlling the growth of pathogens such as fungi in plants. Non-Patent Document 1 or 3 relates to a method for the regioselective or stereoselective synthesis of $\alpha$, $\beta$ -unsaturated amide or ester using a catalyst. Namely, there is no disclosure for an analgesic action in any of these documents. As mentioned above, it is the finding being unknown up to now that a trans-2-decenoic acid derivative to be described or a pharmaceutically acceptable salt thereof in accordance with the analgesic of the present invention has an analgesic action and is effective for pain diseases.

**[Prior Art Documents]**

**[Patent Document]**

Patent Document 1: International Publication No. WO 2009/038110
Patent Document 2: International Publication No. WO 2010/015932

**[Non-Patent Document]**

Non-Patent Document 1: Applied Organometalic Chemistry, 2003, Vol. 17, No. 12, p. 921-931
Non-Patent Document 2: Latin American Journal of Pharmacy, 2008, Vol. 27, No. 6, p. 852-858
Non-Patent Document 3: Synthesis, 2009, No. 15, p. 2634-2645

[Summary of the Invention]

[Problems to be solved by the Invention]

An object of the present invention is to provide an analgesic having an excellent effect.

**[Means for Solving the Problems]**

As a result of intensive studies, the present inventors have found that a trans-2-decenoic acid derivative or a pharmaceutically acceptable salt thereof described below has an excellent analgesic effect and accomplished the present invention. Thus the present invention is as follows.

[1] An analgesic containing at least one member selected from a trans-2-decenoic acid derivative represented by the following formula (1) and a pharmaceutically acceptable salt thereof as an active ingredient.

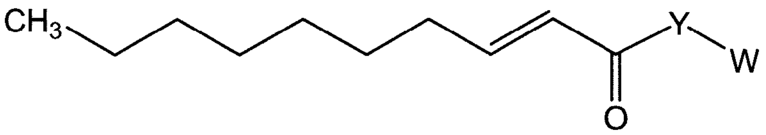

(1)

[In the formula,
Y is -O-, -NR- or -S-;
W is W1 when Y is -O-, W2 when Y is -NR- or W3 when Y is -S-;

(1) W1 is dialkylaminoalkyl group, alkylthioalkyl group, alkoxyalkyl group, dialkoxyalkyl group or dialkylaminoalkoxyalkyl group;
(2-1) W2 is hydrogen atom, alkyl group or dialkylaminoalkyl group when R is dialkylaminoalkyl group;
(2-2) W2 is alkyl group which is same as or different from R when R is alkyl group; or
(2-3) W2 is alkyl group, cycloalkyl group, pyrrolidinealkyl group, phenyl group or phenylalkyl group when R is hydrogen atom; and
(3) W3 is alkyl group, cycloalkyl group, phenylalkyl group or dialkylaminoalkyl group.]

[2] The analgesic according to [1], wherein Y is -O- and W1 is dialkylaminoalkyl group, alkylthioalkyl group, alkoxyalkyl group, dialkoxyalkyl group or dialkylaminoalkoxyalkyl group.
[3] The analgesic according to [1], wherein Y is -NR-.
[4] The analgesic according to [3], wherein R is dialkylaminoalkyl group and W2 is hydrogen atom, alkyl group or dialkylaminoalkyl group.
[5] The analgesic according to [3], wherein R is alkyl group and W2 is alkyl group which is same as or different from R.
[6] The analgesic according to [3], wherein R is hydrogen atom and W2 is alkyl group, cycloalkyl group, pyrrolidinealkyl group, phenyl group or phenylalkyl group.
[7] The analgesic according to [1], wherein Y is -S- and W3 is alkyl group, cycloalkyl group, phenylalkyl group or dialkylaminoalkyl group.
[8] The analgesic according to any of [1] to [7], which is a therapeutic agent for arthralgia.
[9] The analgesic according to [8], wherein the arthralgia is the pain caused by osteoarthritis.
[10] The analgesic according to [9], wherein the osteoarthritis is knee osteoarthritis or hip osteoarthritis.
[11] The analgesic according to any of [1] to [10], which is an injectable preparation.
[12] The analgesic according to any of [1] to [10], which is an oral preparation.
[13] The analgesic according to [11] or [12], wherein the injectable preparation or the oral preparation is a cyclodextrin inclusion complex.
[14] The analgesic according to any of [1] to [10], which is an external preparation.
[15] The analgesic according to [14], wherein the external preparation is a patch preparation.
[16] The trans-2-decenoic acid derivative or the pharmaceutically acceptable salt thereof according to any of [1] to [7] for use in the therapy of pain diseases.
[17] A method for treating pain diseases, comprising administering at least one member selected from the trans-2-decenoic acid derivative and the pharmaceutically acceptable salt thereof according to any of [1] to [7] in effective dose to a patient suffering from pain diseases.
[18] Use of the trans-2-decenoic acid derivative or the pharmaceutically acceptable salt thereof according to any of [1] to [7] in the manufacture of a drug for the therapy of pain diseases.

**[Advantages of the Invention]**

The trans-2-decenoic acid derivative or the pharmaceutically acceptable salt thereof in accordance with the analgesic of the present invention is a compound having an excellent analgesic action and is very useful as a drug for the therapy of various pain diseases including the pain by arthralgia such as osteoarthritis and so on.

**[Mode for Carrying Out the Invention]**

The present invention relates to an analgesic containing at least one member selected from a trans-2-decenoic acid derivative represented by the following formula (1) and a pharmaceutically acceptable salt thereof as an active

ingredient. The compound represented by the following formula (1) is disclosed in the specification of the international application PCT/JP/2011/75228. The said compound can be synthesized by a method to be hereinafter described. If necessary, the detailed synthesis method is available in the international publication of the above international application.

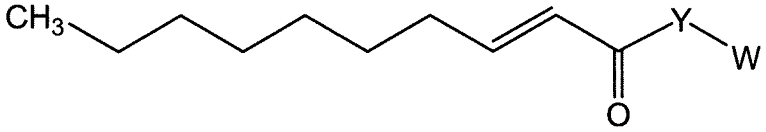 (1)

[In the formula,
Y is -O-, -NR- or -S-;
W is W1 when Y is -O-, W2 when Y is -NR- or W3 when Y is -S-;

(1) W1 is dialkylaminoalkyl group, alkylthioalkyl group, alkoxyalkyl group, dialkoxyalkyl group or dialkylaminoalkoxyalkyl group;
(2-1) W2 is hydrogen atom, alkyl group or dialkylaminoalkyl group when R is dialkylaminoalkyl group;
(2-2) W2 is alkyl group which is same as or different from R when R is alkyl group; or
(2-3) W2 is alkyl group, cycloalkyl group, pyrrolidinealkyl group, phenyl group or phenylalkyl group when R is hydrogen atom; and
(3) W3 is alkyl group, cycloalkyl group, phenylalkyl group or dialkylaminoalkyl group.]

The "alkyl" in "aminoalkyl" when W1' is "dialkylaminoalkyl group" in the substituent of the above formula (1) is any kind of an alkyl group, preferably a linear or branched alkyl group having 1 to 10 carbon(s) such as methyl, ethyl, propyl, isopropyl, butyl, isobutyl, sec-butyl, tert-butyl, pentyl, isopentyl, neopentyl, tert-pentyl, dimethylpropyl, hexyl, isohexyl, heptyl, isoheptyl, octyl, isooctyl, nonyl, isononyl, decyl or isodecyl and, more preferably, a linear or branched alkyl group having 1 to 6 carbon(s).

Each of the "alkyl" when both R' and W2' are alkyl group, which may be same or different, is any kind of an alkyl group, preferably a liner or branched alkyl group having 1 to 10 carbon(s) such as methyl, ethyl, propyl, isopropyl, butyl, isobutyl, sec-butyl, tert-butyl, pentyl, isopentyl, neopentyl, tert-pentyl, hexyl, isohexyl, heptyl, isoheptyl, octyl, isooctyl, nonyl, isononyl, decyl or isodecyl and, more preferably, a liner or branched alkyl group having 1 to 7 carbon(s).

The "alkyl" when R' is hydrogen atom and W2' is alkyl group is any kind of an alkyl group, preferably a liner or branched alkyl group having 1 to 10 carbon(s) such as methyl, ethyl, propyl, isopropyl, butyl, isobutyl, sec-butyl, tert-butyl, 2-metylbutyl, pentyl, isopentyl, neopentyl, tert-pentyl, hexyl, isohexyl, heptyl, isoheptyl, 1-propylbutyl, octyl, isooctyl, 1-ethylhexyl, 1,1,3,3-tetramethylbutyl, nonyl, isononyl, decyl or isodecyl and, more preferably, a liner or branched alkyl group having 1 to 8 carbon(s).

The "alkyl" when W3' is alkyl group is any kind of an alkyl group, preferably a liner or branched alkyl group having 1 to 12 carbon(s) such as methyl, ethyl, propyl, isopropyl, butyl, isobutyl, sec-butyl, tert-butyl, pentyl, isopentyl, neopentyl, tert-pentyl, hexyl, isohexyl, heptyl, isoheptyl, octyl, isooctyl, nonyl, isononyl, decyl, isodecyl, undecyl, isoundecyl, dodeyl or isododecyl and, more preferably, a liner or branched alkyl group having 4 to 10 carbons.

The "cycloalkyl group" is any kind of a cycloalkyl group, preferably a cycloalkyl group having 3 to 8 carbons such as cyclopropyl, cyclobutyl, cyclopentyl, cyclohexyl, cycloheptyl or cyclooctyl and, more preferably, a cycloalkyl group having 5 or 6 carbons.

The "alkyl" which is other than the above-specified ones in the substituent of the above formula (1) is any kind of an alkyl group, preferably a liner or branched alkyl group having 1 to 4 carbon(s) such as methyl, ethyl, propyl, isopropyl, butyl, isobutyl, sec-butyl or tert-butyl.

The "alkoxy" in the substituent of the above formula (1) is any kind of an alkoxy group, preferably a liner or branched alkoxy group having 1 to 4 carbon(s) such as methoxy, ethoxy, propoxy, isopropoxy, butoxy, isobutoxy, sec-butoxy or tert-butoxy.

The compound of the present invention represented by the formula (1) is able to be produced using trans-2-decenoic acid as a material, for example, as shown in the following reaction formulae.

[Reaction formulae]

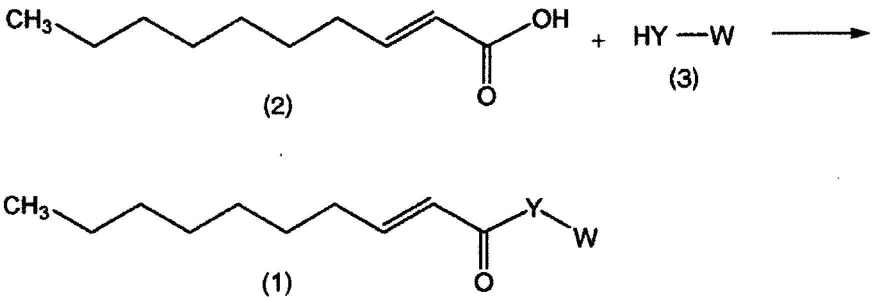

(2) (3) (1)

(In the formulae, Y and W are the same as those mentioned already.)

The compound represented by the formula (1) is able to be produced by subjecting the compound represented by the formula (2) and the compound represented by the formula (3) to a dehydration-condensation. The dehydration-condensation reaction may adopt the conventionally known methods.

For example, the compound represented by the formula (2) may be made to react with the compound represented by the formula (3) in the presence of an appropriate condensing agent (such as dicyclohexylcarbodiimide (DCC) or N-(3-dimethylaminopropyl)-N'-ethylcarbodiimide·HCl). The reaction may be usually carried out in a common solvent (such as dichloromethane). Usually, the using amount of the compound represented by the formula (3) is 0.5 to 2 mol (preferably, 1 to 1.5 mol) to 1 mol of the compound represented by the formula (2).

Alternatively, the compound represented by the formula (2) may be, for example, once converted to a carboxylic halide and then made to react with the compound represented by the formula (3) in the presence or absence of a base. Conversion to the carboxylic halide may be carried out, for example, using a halogenating agent such as thionyl chloride, sulfyryl chloride, phosphorus trichloride, phosphorus pentachloride, oxalyl chloride or phosphoric acid trichloride. Examples of the base include triethylamine and pyridine. Usually, the using amount of the compound represented by the formula (3) is 0.5 to 2 mol (preferably, 1 to 1.5 mol) to 1 mol of the compound represented by the formula (2). When a base is used, the using amount of the base is usually about 1 to 5 mol to 1 mol of the compound represented by the formula (2).

After finishing the above reaction, the aimed compound is able to be produced using the known purifying and isolating operations (such as extraction, chromatography, distillation or recrystallization).

The compound represented by the formula (1) includes not only and naturally the above-mentioned free form but also the forms of salt, solvate and prodrug. In forming the salt, the form of a pharmaceutically acceptable salt is advantageous in using as a pharmaceutical agent. Examples of the salt include that with an inorganic acid such as phosphoric acid, hydrochloric acid, sulfuric acid or nitric acid and that with an organic acid such as citric acid, tartaric acid, lactic acid or glycolic acid. These salts can be produced by known methods, for example, a solution containing equimolar amounts of a trans-2-decenoic acid derivative represented by the formula (1) and an organic acid such as citric acid is prepared and a salt can be obtained as crystal by removing a solvent. Examples of the solvate include hydrate and a solvate with alcohol. When the compound of the present invention represented by the formula (1) contains asymmetric carbon(s), it includes various kinds of isomers such as optical isomer, racemic substance or diastereomer. When the compound of the present invention becomes crystals, it also includes various kinds of crystal forms (crystal polymorphism) being able to be formed thereby.

Examples of the compound produced as such are shown in Tables 1 and 2. When each compound is referred to hereinafter, the compound number mentioned in the tables is used.

[Table 1]

| Compound No. | Compound Name | Structural Formula |
|---|---|---|
| 1 | (*E*)-2-(dimethylamino)ethyl dec-2-enoate | 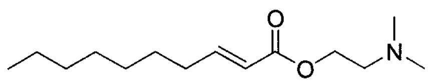 |
| 2 | (*E*)-3-(dimethylamino)propyl dec-2-enoate | 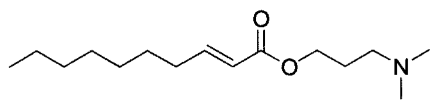 |

(continued)

| Compound No. | Compound Name | Structural Formula |
|---|---|---|
| **3** | (*E*)-1-(dimethylamino)propan-2-yl dec-2-enoate | 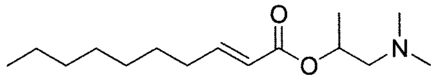 |
| **4** | (*E*)-4-(dimethylamino)butyl dec-2-enoate | 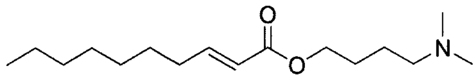 |
| **5** | (*E*)-3-(dimethylamino)-2,2-dimethylpropyl dec-2-enoate | 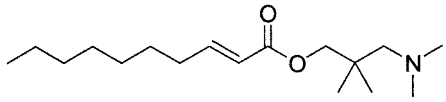 |
| **6** | (*E*)-2-(diethylamino)ethyl dec-2-enoate | 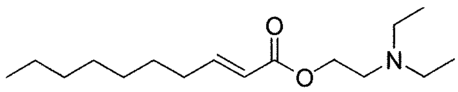 |
| **7** | (*E*)-6-(dimethylamino)hexyl dec-2-enoate | 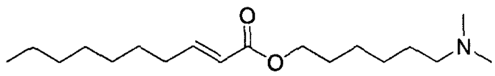 |
| **8** | (*E*)-2-(isopropylthio)ethyl dec-2-enoate | 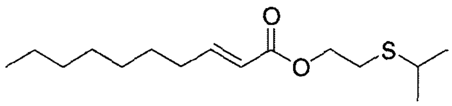 |
| **9** | (*E*)-2-methoxyethyl dec-2-enoate | 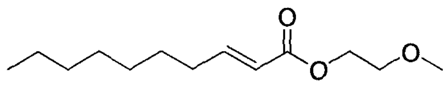 |
| **10** | (*E*)-2-ethoxyethyl dec-2-enoate | 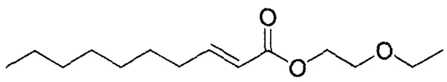 |
| **11** | (*E*)-1,3-diethoxy-2-propyl dec-2-enoate | 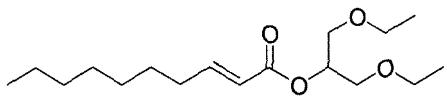 |
| **12** | (*E*)-2-(2-(dimethylamino)ethoxy)ethyl dec-2-enoate | 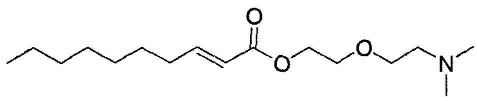 |
| **13** | (*E*)-2-(2-(diethylamino)ethoxy)ethyl dec-2-enoate | 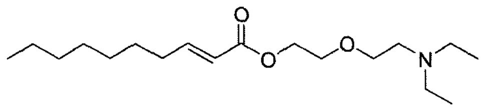 |
| **14** | (*E*)-3-(2-(diethylamino)ethoxy)propyl dec-2-enoate | 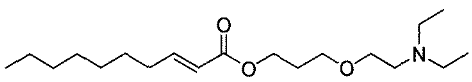 |
| **15** | (*E*)-*N*-methyl dec-2-enamide | 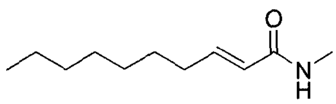 |
| **16** | (*E*)-*N*-ethyl dec-2-enamide | 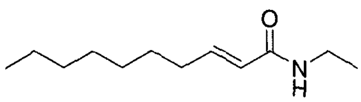 |
| **17** | (*E*)-*N*-butyl dec-2-enamide | 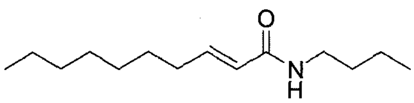 |

[Table 2]

| Compound No. | Compound Name | Structural Formula |
|---|---|---|
| **18** | (*E*)-*N*-isobutyl dec-2-enamide | 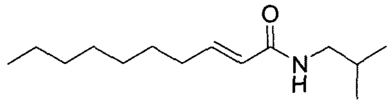 |
| **19** | (*E*)-*N*-pentyl dec-2-enamide | 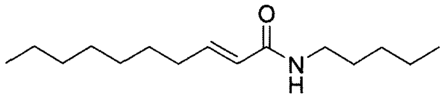 |
| **20** | (*E*)-*N*-isopentyl dec-2-enamide | 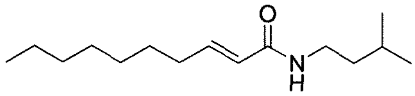 |
| **21** | *(E)-N-tert* -pentyl dec-2-enamide | 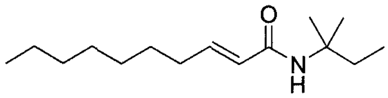 |
| **22** | (*E*)-*N*-hexyl dec-2-enamide | 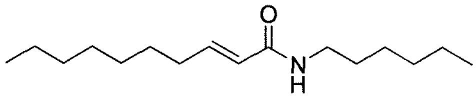 |
| **23** | (*E*)-*N*-heptyl dec-2-enamide | 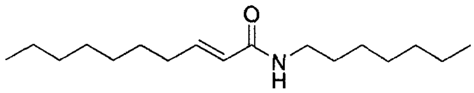 |
| **24** | (*E*)-*N*-(heptan-4-yl) dec-2-enamide | 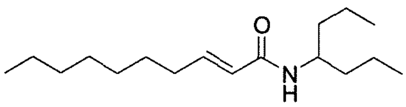 |
| **25** | (*E*)-*N*-(octan-3-yl) dec-2-enamide | 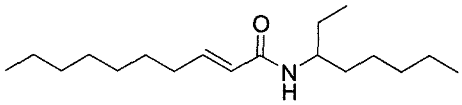 |
| **26** | (*E*)-*N*-(2,4,4-trimethylpentan-2-yl) dec-2-enamide | 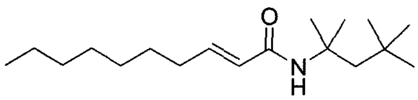 |
| **27** | (*E*)-*N*-cyclohexyl dec-2-enamide | 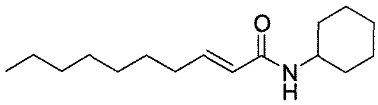 |
| **28** | (*E*)-*N*-phenyl dec-2-enamide | 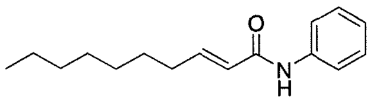 |
| **29** | (*E*)-*N*-phenethyl dec-2-enamide | 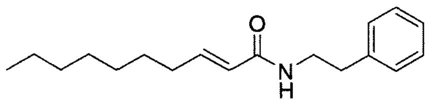 |
| **30** | (*E*)-*N*-(2-pyrrolidin-1-ylethyl) dec-2-enamide) | 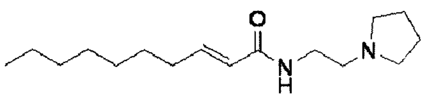 |
| **31** | (*E*)-*N,N*-diethyl dec-2-enamide | 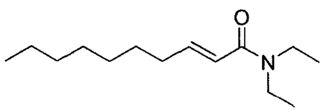 |

(continued)

| Compound No. | Compound Name | Structural Formula |
|---|---|---|
| **32** | (*E*)-*N,N*-dibutyl dec-2-enamide | 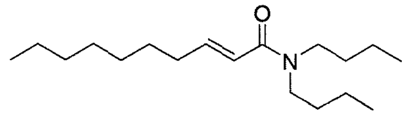 |
| **33** | (*E*)-*N,N*-dipentyl dec-2-enamide | 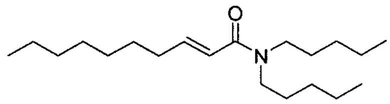 |

[Table 3]

| Compound No. | Compound Name | Structural Formula |
|---|---|---|
| **34** | (*E*)-*N,N*-dihexyl dec-2-enamide | 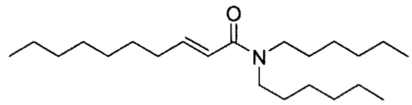 |
| **35** | (*E*)-*N*-ethyl-N-heptyl dec-2-enamide | 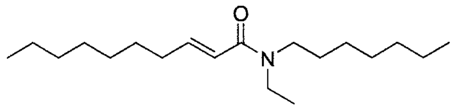 |
| **36** | (*E*)-*N*-2-(dimethylamino)ethyl dec-2-enamide | 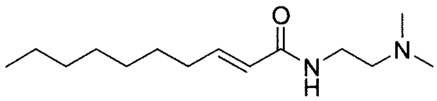 |
| **37** | (*E*)-*N*-2-(diethylamino)ethyl dec-2-enamide | 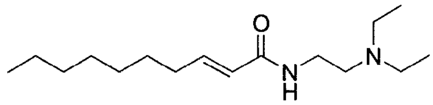 |
| **38** | (*E*)-*N*-3-(dimethylamino)propyl dec-2-enamide | 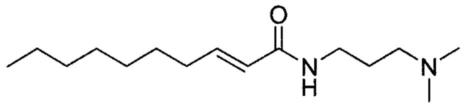 |
| **39** | *(E)-N*-3-(diethylamino)propyl dec-2-enamide | 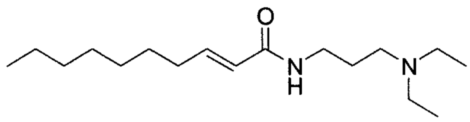 |
| **40** | (*E*)-*N*-2-(diisopropylamino)ethyl dec-2-enamide | 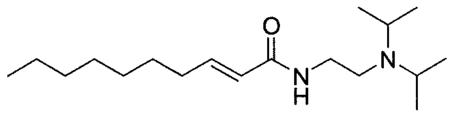 |
| **41** | (*E*)-*N*-2-(dibutylamino)ethyl dec-2-enamide | 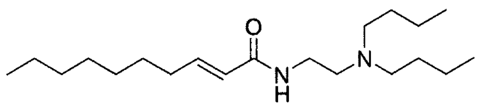 |
| **42** | (*E*)-*N*-(2-(dimethylamino)ethyl)-N-methyl dec-2-enamide | 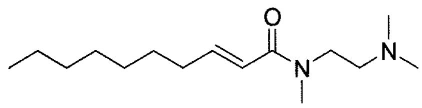 |
| **43** | (*E*)-*N*-(2-(dimethylamino)ethyl)-N-ethyl dec-2-enamide | 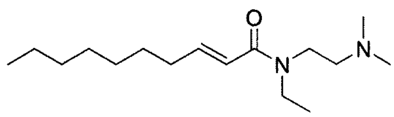 |

(continued)

| Compound No. | Compound Name | Structural Formula |
|---|---|---|
| **44** | (*E*)-*N*-(2-(diethylamino)ethyl)-N-ethyl dec-2-enamide | 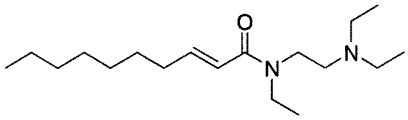 |
| **45** | (*E*)-*N,N*-bis(2-(dimethylamino)ethyl) dec-2-enamide | 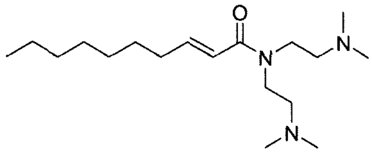 |

[Table 4]

| Compound No. | Compound Name | Structural Formula |
|---|---|---|
| **46** | (*E*)-*N,N*-bis(2-(diethylamino)ethyl) dec-2-enamide | 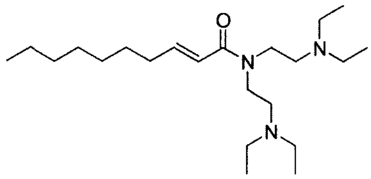 |
| **47** | (*E*)-*N,N*-bis(3-(dimethylamino)propyl) dec-2-enamide | 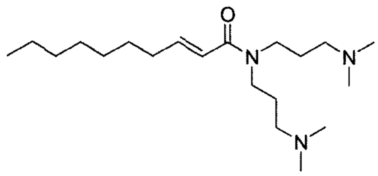 |
| **48** | (*E*)-*S*-pentyl dec-2-enethioate | 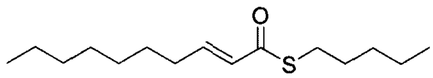 |
| **49** | (*E*)-*S*-isopentyl dec-2-enethioate | 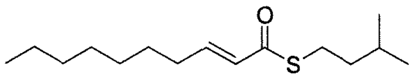 |
| **50** | (*E*)-*S*-hexyl dec-2-enethioate | 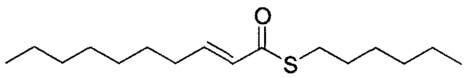 |
| **51** | (*E*)-*S*-heptyl dec-2-enethioate | 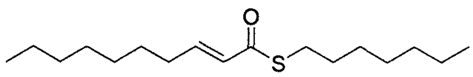 |
| **52** | (*E*)-*S*-decyl dec-2-enethioate | 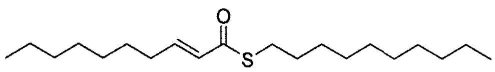 |
| **53** | (*E*)-*S*-cyclopentyl dec-2-enethioate | 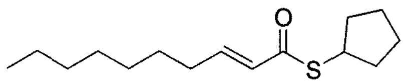 |
| **54** | (*E*)-*S*-phenethyl dec-2-enethioate | 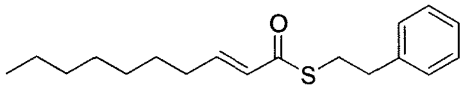 |
| **55** | (*E*)-*S*-2-(dimethylamino)ethyl dec-2-enethioate | 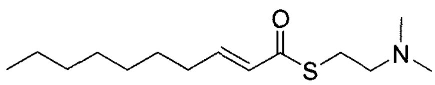 |

(continued)

| Compound No. | Compound Name | Structural Formula |
|---|---|---|
| **56** | (*E*)-*S*-2-(diethylamino)ethyl dec-2-enethioate | 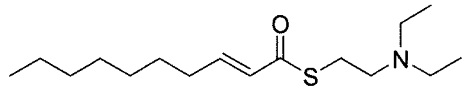 |

The analgesic of the present invention contains at least one member selected from a trans-2-decenoic acid derivative and a pharmaceutically acceptable salt thereof as an active ingredient and is useful as a prophylactic or therapeutic agent for various pain diseases. Examples of the pain diseases, which may be any kind of one, include arthralgia such as the pain by osteoarthritis (e.g., knee osteoarthritis and hip osteoarthritis) or by rheumatoid arthritis.

The trans-2-decenoic acid derivative or the pharmaceutically acceptable salt thereof of the present invention can be made into a pharmaceutical preparation in various dosage forms (such as oral, injectable and external preparations) by appropriately combining with an appropriate pharmaceutical carrier or diluent. The analgesic of the present invention may be also a combination drug in which the compound as its active ingredient is combined with other pharmaceutically active ingredient(s). Further, the analgesic of the present invention may be made into a preparation as a cyclodextrin inclusion complex or the like. As a result, there may be the cases where enhancement of pharmacological activity, improvement in stability, prolonged action, easy handling, etc. can be achieved. An inclusion complex can be formed by, for example, mixing the compound as active ingredient of the analgesic of the present invention with α.-, β- or γ-cyclodextrin.

When the analgesic of the present invention is made into an oral preparation, it is possible to make into tablet, powder, granule or capsule preparation by means of such a formulation where the compound of the present invention is appropriately combined with an appropriate additive such as excipient, binder, disintegrator, lubricant, extender, wetting agent, buffer, preservative or flavoring. In making into an injectable preparation, it is possible to make into an injectable preparation by addition of stabilizer, preservative, isotonic agent or the like to a solution or suspension containing the compound according to the analgesic of the present invention. In making into an external preparation, it is possible to make into an external preparation such as patch preparation, gel preparation, ointment, cream preparation or the like. Thus, the compound according to the analgesic of the present invention is, for example, mixed with, melted in or emulsified in an appropriate base and, in the case of a patch preparation, the above is spread and applied onto a support. In the case of a patch preparation, a gel preparation or the like, it can be made, for example, into a composition using an organogelling agent. Incidentally, depending upon the dosage form of each external preparation, a commonly used preservative, antioxidant, flavoring agent, adhesive or the like may be appropriately selected and added to a formulation.

Adequate dose of the analgesic of the present invention may be appropriately increased or decreased by taking dose regimen, age, sex, symptom in a patient, etc. into consideration and, may be generally administered in an amount of from 1 to 1,000 mg or, preferably, 5 to 300 mg, for adult, at ounce or in several divided administrations per day.

**[Examples]**

Hereinafter, an example of results of a pharmacological test concerning novel pharmacological activity of the trans-2-decenoic acid derivative or the pharmaceutically acceptable salt thereof according to the present invention, that is, an analgesic effect, is described. The present invention is not intended to be limited to the descriptions in Examples.

Pharmacological Test I: Analgesic action to osteoarthritis model rats

There were conducted the following experiments for testing the analgesic action of the analgesic of the present invention using the osteoarthritis (OA) rats induced by sodium monoiodoacetate (MIA) which were model animals for OA.

(1) Preparation of MIA-induced OA rats

A 50% reaction threshold value to the mechanical stimulus of male Wistar rats of six weeks age was measured (the measuring method will be mentioned later) and a normal control group was selected. MIA prepared by saline was administered in a single dose of 300 μg/50 μL into right knee joint of the rats except the normal control group while 50 μL of saline was administered into left knee joint whereupon the MIA-induced OA rats were prepared. To the normal control group, 50 μL of saline was administered into the joints of both knees.

(2) Grouping

With regard to the male Wistar rats of six weeks age used as experimental animals except the normal control group, their 50% reaction threshold values to the mechanical stimulus (the measuring method will be mentioned later) and body weights were measured after 24 days from the administration of MIA mentioned in (1) and then 3 groups each comprising six rats [a normal control group, an onset control group and a test drug-administered group] were organized.

(3) Administration of test drug

A test drug solution (100 μg/mL) using the compound according to the analgesic of the present invention as a test drug was prepared using a phosphate buffered saline (PBS) containing 0.1 vol% of dimethyl sulfoxide (DMSO).

Immediately after the grouping (after 14 days from the administration of MIA), a test drug solution was intraperitoneally administered in a single dose of 200 μg/kg to a test drug-administered group. Meanwhile, to the normal control group and the onset control group, PBS containing 0.1 vol% of DMSO was intraperitoneally administered in a single dose.

(4) Result of measurement of the 50% reaction threshold values to the mechanical stimulus (von Frey test)

The three groups of rats of the above (2) were placed in a transparent acrylic cage with a wire-meshed floor and habituated for about three minutes and the 50% reaction threshold values to the mechanical stimulus were measured after 1 hour from the administration of a test drug. The measurement was conducted using von Frey filaments (manufactured by North Coast Medical Inc.) in accordance with the methods of Chaplan, et al. (Journal of Neuroscience Methods, vol. 53, no. 1, pages 55 to 63, 1994) and Dixon, et al. (Annual Review of Pharmacology and Toxicology, vol. 20, pages 441 to 462, 1980). In eight filaments [stimulus loads (g): 0.4, 0.6, 1.0, 2.0, 4.0, 6.0, 8.0 and 15.0], the test was started as from the filament of 2.0 g, the filament was vertically attached to the sole for 2 to 3 seconds with such a force that the filament was lightly bent and the case where the hind limb showed an escape reaction was called a positive reaction. The case where the rat escaped at the instance of removing the filament was also called positive. When the positive reaction was noted, stimulus was conducted similarly using a filament of one rank weaker while, when no reaction was noted, stimulus was conducted similarly using a filament of one rank stronger and the point when the reaction changed from negative to positive or from positive to negative was called the first two reactions. After that, stimulus was conducted for continuous four times by the same up-down method. A 50% reaction threshold value to the mechanical stimulus was measured using the reaction to the six stimuli in total and then (mean value) ± (standard error) for each group was calculated. Incidentally, when stimulus reached by that of 15.0 g without positive reaction or, when positive reaction continued to 0.4 g, then 15.0 g or 0.25 g was adopted as each threshold value, respectively.

With regard to the 50% reaction threshold value after 1 hour from administration of a test drug, a recovery rate (%) of the 50% reaction threshold value was calculated by the following expression in which 15 was adopted as the normal threshold value.

Recovery rate (%) of 50% reaction threshold value = {[(50% reaction threshold value after 1 hour from administration of test drug) - (50% reaction threshold value before administration of test drug)] ÷ [(Normal threshold value) - (50% reaction threshold value before administration of test drug)]} × 100

[Table 5]

| Test drug | Recovery rate of 50% reaction threshold value (%) |
|---|---|
| Compound 1 | 25.5 |
| Compound 8 | 28.9 |
| Compound 9 | 13.0 |

(continued)

| Test drug | Recovery rate of 50% reaction threshold value (%) |
|---|---|
| Compound 18 | 49.0 |
| Compound 22 | 44.3 |
| Compound 27 | 5.8 |
| Compound 37 (Citrate) | 45.1 |
| Compound 44 (Citrate) | 21.7 |
| Compound 45 | 10.2 |
| Compound 50 | 15.6 |
| Compound 56 | 21.8 |

As will be apparent from Table 5, the analgesic of the present invention showed an excellent suppressive effect to hyperalgesia of OA induced by the administration of MIA.

**[Industrial Applicability]**

As shown in the results of the above pharmacological tests, the analgesic of the present invention shows excellent analgesic effect and suppressive effect for hyperalgesia in animal experiments using MIA-induced OA rats which are an OA model. Accordingly, the analgesic of the present invention is highly useful as a prophylactic or therapeutic agent for various pain diseases such as the pain caused by OA

**Claims**

1. An analgesic containing at least one member selected from a trans-2-decenoic acid derivative represented by the following formula (1) and a pharmaceutically acceptable salt thereof as an active ingredient.

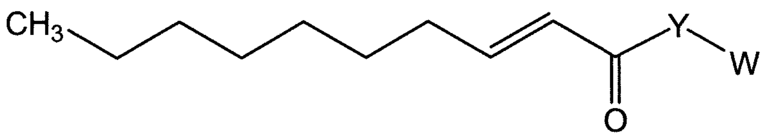

(1)

[In the formula,
Y is -O-, -NR- or -S-;
W is W1 when Y is -O-, W2 when Y is -NR- or W3 when Y is -S-;

(1) W1 is dialkylaminoalkyl group, alkylthioalkyl group, alkoxyalkyl group, dialkoxyalkyl group or dialkylaminoalkoxyalkyl group;
(2-1) W2 is hydrogen atom, alkyl group or dialkylaminoalkyl group when R is dialkylaminoalkyl group;
(2-2) W2 is alkyl group which is same as or different from R when R is alkyl group; or
(2-3) W2 is alkyl group, cycloalkyl group, pyrrolidinealkyl group, phenyl group or phenylalkyl group when R is hydrogen atom; and
(3) W3 is alkyl group, cycloalkyl group, phenylalkyl group or dialkylaminoalkyl group.]

2. The analgesic according to claim 1, wherein Y is -O- and W1 is dialkylaminoalkyl group, alkylthioalkyl group, alkoxyalkyl group, dialkoxyalkyl group or dialkylaminoalkoxyalkyl group.

3. The analgesic according to claim 1, wherein Y is -NR-.

4. The analgesic according to claim 3, wherein R is dialkylaminoalkyl group and W2 is hydrogen atom, alkyl group or dialkylaminoalkyl group.

**5.** The analgesic according to claim 3, wherein R is alkyl group and W2 is alkyl group which is same as or different from R.

**6.** The analgesic according to claim 3, wherein R is hydrogen atom and W2 is alkyl group, cycloalkyl group, pyrrolidinealkyl group, phenyl group or phenylalkyl group.

**7.** The analgesic according to claim 1, wherein Y is -S- and W3 is alkyl group, cycloalkyl group, phenylalkyl group or dialkylaminoalkyl group.

**8.** The analgesic according to any of claims 1 to 7, which is a therapeutic agent for arthralgia.

**9.** The analgesic according to claim 8, wherein the arthralgia is the pain caused by osteoarthritis.

**10.** The analgesic according to claim 9, wherein the osteoarthritis is knee osteoarthritis or hip osteoarthritis.

**11.** The analgesic according to any of claims 1 to 10, which is an injectable preparation.

**12.** The analgesic according to any of claims 1 to 10, which is an oral preparation.

**13.** The analgesic according to claim 11 or 12, wherein the injectable preparation or the oral preparation is a cyclodextrin inclusion complex.

**14.** The analgesic according to any of claims 1 to 10, which is an external preparation.

**15.** The analgesic according to claim 14, which is a patch preparation.

## INTERNATIONAL SEARCH REPORT

International application No.
PCT/JP2013/062374

A. CLASSIFICATION OF SUBJECT MATTER
*A61K31/16*(2006.01)i, *A61K9/08*(2006.01)i, *A61K9/20*(2006.01)i, *A61K9/70*(2006.01)i, *A61K31/231*(2006.01)i, *A61K31/265*(2006.01)i, *A61K47/40*(2006.01)i, *A61P19/02*(2006.01)i, *A61P29/00*(2006.01)i

According to International Patent Classification (IPC) or to both national classification and IPC

B. FIELDS SEARCHED

Minimum documentation searched (classification system followed by classification symbols)
A61K31/16, A61K9/08, A61K9/20, A61K9/70, A61K31/231, A61K31/265, A61K47/40, A61P19/02, A61P29/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CAplus/REGISTRY/MEDLINE/EMBASE/BIOSIS(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| A | Janusz JM, et al, Vanilloids. 1. Analogs of capsaicin with antinociceptive and antiinflammatory activity., J Med Chem., 1993, vol.36, no.18, p.2595-2604 | 1-15 |
| A | Yang XY, et al, 10-Hydroxy-2-decenoic acid from Royal jelly: a potential medicine for RA., J Ethnopharmacol., 2010, vol.128, no. 2, p.314-321 | 1-15 |
| A | Concellón JM, et al, Stereospecific cyclopropanation of highly substituted C-C double bonds promoted by CrCl2. Stereoselective synthesis of cyclopropanecarboxamides and cyclopropyl ketones, Organic Letters (2007), 9(16), 2981-2984 | 1-15 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 July, 2013 (26.07.13) | 06 August, 2013 (06.08.13) |
| Name and mailing address of the ISA/ Japanese Patent Office | Authorized officer |
| Facsimile No. | Telephone No. |

Form PCT/ISA/210 (second sheet) (July 2009)

INTERNATIONAL SEARCH REPORT

International application No.
PCT/JP2013/062374

C (Continuation). DOCUMENTS CONSIDERED TO BE RELEVANT

| Category* | Citation of document, with indication, where appropriate, of the relevant passages | Relevant to claim No. |
|---|---|---|
| A | JP 2007-510634 A (Symrise GmbH & Co. KG.), 26 April 2007 (26.04.2007), & US 2007/0202188 A1 & EP 1682489 A & WO 2005/044778 A1 | 1-15 |
| A | US 2009/0124701 A1 (SYMRISE GmbH & Co. KG), 14 May 2009 (14.05.2009), & EP 2058297 A1 | 1-15 |
| A | Ando K, Preparations of Z-α,β-unsaturated amides by using Horner-Wadsworth-Emmons reagents, (diphenylphosphono)acetamides, Synlett, 2001, no.8, p.1272-1274 | 1-15 |
| P,A | WO 2012/060396 A1 (Nagoya Industrial Science Research Institute), 10 May 2012 (10.05.2012), a whole article & JP 5044059 B & AU 2011324414 A & CA 2815964 A | 1-15 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009038110 A
- WO 2010015932 A
- JP 2011075228 W


### Non-patent literature cited in the description


- *Applied Organometalic Chemistry,* 2003, vol. 17 (12), 921-931
- *Latin American Journal of Pharmacy,* 2008, vol. 27 (6), 852-858
- *Synthesis,* 2009, vol. 15, 2634-2645
- **CHAPLAN et al.** *Journal of Neuroscience Methods,* 1994, vol. 53 (1), 55-63
- **DIXON et al.** *Annual Review of Pharmacology and Toxicology,* 1980, vol. 20, 441-462